(19) Europäisches Patentamt
European Patent Office
Office européen des brevets

(11) **EP 2 486 901 A1**

(12) **EUROPEAN PATENT APPLICATION**
published in accordance with Art. 153(4) EPC

(43) Date of publication:
**15.08.2012 Bulletin 2012/33**

(51) Int Cl.:
**A61F 13/15** (2006.01)  **A61F 13/49** (2006.01)
**A61F 13/53** (2006.01)

(21) Application number: **10821927.0**

(22) Date of filing: **01.10.2010**

(86) International application number:
**PCT/JP2010/067225**

(87) International publication number:
**WO 2011/043256 (14.04.2011 Gazette 2011/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2009 JP 2009235382**

(71) Applicant: **Sumitomo Seika Chemicals Co. Ltd. Kako-gun, Hyogo 675-0145 (JP)**

(72) Inventors:
• **MARUO, Junichi**
  **Hyogo 672-8076 (JP)**
• **TSUNO, Syuji**
  **Hyogo 675-0145 (JP)**
• **MATSUSHITA, Hideki**
  **Hyogo 672-8076 (JP)**
• **HANDA, Masayoshi**
  **Hyogo 672-8076 (JP)**

(74) Representative: **Jackson, Martin Peter**
   **J A Kemp**
   **14 South Square**
   **Gray's Inn**
   **London WC1R 5JJ (GB)**

(54) **WATER ABSORBENT SHEET**

(57)    A water-absorbent sheet comprising a structure in which an absorbent layer containing a water-absorbent resin and an adhesive is sandwiched with nonwoven fabrics from an upper side and a lower side of the absorbent layer, wherein the water-absorbent sheet has a laminate structure in which the absorbent layer is fractionated into a primary absorbent layer and a secondary absorbent layer with a breathable fractionating layer, and wherein at least one sheet out of the nonwoven fabrics is a spunbond nonwoven fabric, and at least a nonwoven fabric at a lower side out of the nonwoven fabrics is a hydrophilic nonwoven fabric. The water-absorbent sheet of the present invention exhibits some effects that a water-absorbent sheet is capable of accomplishing avoidance of gel blocking phenomenon and liquid leakage, while obtaining basic properties as a water-absorbent sheet at a high level, by using specified nonwoven fabrics, and further providing a hydrophilic nonwoven fabric as a lower side of a nonwoven fabric of the absorbent layer of a water-absorbent sheet, even for a water-absorbent sheet containing a very small amount of pulps and being thinner than a conventional product.

[Figure 1]

FIG. 1

EP 2 486 901 A1

**Description**

TECHNICAL FIELD

The present invention relates to a water-absorbent sheet which can be used in the fields of hygienic materials and the like. More specifically, the present invention relates to a water-absorbent sheet containing a very small amount of pulp, which can be suitably used in absorbent articles, such as disposable diapers and incontinence pads, having high absorbent properties even when the water-absorbent sheet is thinner than a conventional product, and having high shape retaining ability. In addition, the present invention relates to an absorbent article using the water-absorbent sheet.

BACKGROUND ART

Absorbent articles represented by disposable diapers or the like have a structure in which an absorbent material for absorbing a liquid such as a body liquid is sandwiched with a flexible liquid-permeable surface sheet (top sheet) positioned on a side contacting a body and a liquid-impermeable backside sheet (back sheet) positioned on a side opposite to that contacting the body.

Conventionally, there have been increasing demands for thinning and light-weighing of absorbent articles, from the viewpoint of designing property and convenience upon carrying, and efficiency upon distribution. Further, in the recent years, there have been growing needs for so-called eco-friendly intentions, in which resources are effectively utilized so that use of natural materials that require a long time to grow such as trees is avoided as much as possible, from the viewpoint of environmental protection. Conventionally, a method for thinning that is generally carried out in absorbent articles is a method of reducing hydrophilic fibers such as crushed pulp of a wood material, which has a role of fixing a water-absorbent resin in an absorbent material, while increasing a water-absorbent resin.

An absorbent material in which a water-absorbent resin is used in a large amount with a lowered proportion of a hydrophilic fiber is preferred in thinning, from the viewpoint of reducing bulky hydrophilic fibers while retaining a liquid. However, when distribution or diffusion of a liquid upon actually using in an absorbent article such as disposable diapers is considered, there is a disadvantage that if a large amount of the water-absorbent resin is formed into a soft gel-like state by absorption, a so-called "gel-blocking phenomenon" takes place, whereby liquid diffusibility is markedly lowered and a liquid permeation rate of the absorbent material is slowed down. This "gel-blocking phenomenon" is a phenomenon in which especially when an absorbent material in which water-absorbent resins are highly densified absorbs a liquid, water-absorbent resins existing near a surface layer absorb the liquid to form soft gels that are even more densified near the surface layer, so that a liquid permeation into an internal of an absorbent material is inhibited, thereby making the internal of the water-absorbent resin incapable of efficiently absorbing the liquid.

In view of the above, conventionally, as a means of inhibiting gel blocking phenomenon which takes place by reducing hydrophilic fibers while using a water-absorbent resin in a large amount, for example, proposals such as a method using an absorbent polymer having such properties as specified Saline Flow Conductivity and Performance under Pressure (see Patent Publication 1), and a method using a water-absorbent resin prepared by heat-treating a specified water-absorbent resin precursor with a specified surface crosslinking agent (see Patent Publication 2) have been made.

However, in these methods, the absorbent properties as absorbent materials in which water-absorbent resins are used in large amounts are not satisfactory. In addition, there arise some problems that the water-absorbent resin is subjected to be mobile before use or during use because hydrophilic fibers that play a role of fixing the water-absorbent resin are reduced. The absorbent material in which the localization of the absorbent resin takes place is more likely to cause gel-blocking phenomenon.

Further, in an absorbent material of which hydrophilic fibers that contribute to retention of the form are reduced has a lowered strength as an absorbent material, deformation in shapes such as twist-bending or tear before or after the absorption of a liquid is likely to take place. In an absorbent material with deformation in shapes, liquid diffusibility has markedly lowered, so that abilities inherently owned by the absorbent material cannot be exhibited. In order to try to avoid such phenomena, a ratio of hydrophilic fibers and a water-absorbent resin would be limited, thereby posing limitations in the thinning of an absorbent article.

In view of the above, in recent years, as a next generation style absorbent material which is capable of increasing a content of a water-absorbent resin while using hydrophilic fibers in an absorbent material as little as possible, studies have been widely made on an absorbent laminate that substantially does not contain hydrophilic fibers in an absorbent layer, a water-absorbent sheet or the like. For example, a method using an absorbent laminate comprising two pieces of nonwoven fabrics, and a reticular layer comprising two, upper and lower layers of hot melt adhesives provided between the nonwoven fabrics, in which the nonwoven fabrics are bonded with the reticular layer (see Patent Publication 3), a method of sealing a water-absorbent polymer between two sheets of meltblown nonwoven fabrics (see Patent Publication 4), a method of interposing water-absorbent polymer particles between a hydrophobic nonwoven fabric and a hydrophilic sheet (see Patent Publication 5), and the like.

However, in a case where hydrophilic fibers are hardly used, the gel blocking phenomenon as mentioned above is likely to take place. Even in a case where gel blocking phenomenon does not take place, a thing that would serve the role of conventional hydrophilic fibers by which a body fluid such as urine is temporarily subjected to water retention and diffusion of the liquid to an overall absorbent material is lacking, so that a liquid leakage is likely to occur in the absorbent laminate, without being able to sufficiently capture the liquid.

Further, when an adhesive is used for retaining the shape of an absorbent laminate, the surface of an absorbent resin is coated with an adhesive, so that absorbent properties are likely to be lowered. Alternatively, an upper side and a lower side of nonwoven fabrics are firmly adhered with an adhesive to confine an water-absorbent resin in a pouched form or the like, so that the absorbent properties inherently owned by the water-absorbent resin are less likely to be exhibited.

When adhesive strength of an absorbent laminate is weakened in order to improve absorbent properties, not only a large amount of the absorbent resin is detached upon handling the laminate, thereby making unfavorable economically, but also the laminate is exfoliated due to deficiency in strength, so that there are some possibilities of loss of commercial values. In other words, if adhesion is strengthened, gel blocking phenomenon or liquid leakage occurs, and if adhesion is weakened, the detachment of a water-absorbent resin and the breaking of the laminate take place, so that an absorbent laminate or a water-absorbent sheet having satisfactory properties is not obtained.

There is also a method of immobilizing a water-absorbent resin to a substrate without using an adhesive, which is, for example, a method of adhering water-absorbent polymer particles in the process of polymerization to a synthetic fibrous substrate to carry out polymerization on the fibrous substrate (see Patent Publication 6), a method of polymerizing a monomer aqueous composition containing acrylic acid and an acrylic acid salt as main components on a nonwoven fabric substrate by means of electron beam irradiation (see Patent Publication 7), and the like.

In these methods, while the synthetic fibrous substrate is penetrated into the polymer particles to be firmly adhered, there are some disadvantages that it is difficult to complete the polymerization reaction in the substrate, so that unreacted monomers and the like remain in the substrate in large amounts.

PRIOR ART PUBLICATIONS

PATENT PUBLICATIONS

Patent Publication 1: Japanese Unexamined Patent Publication No. Hei-9-510889
Patent Publication 2: Japanese Patent Laid-Open No. Hei-8-057311
Patent Publication 3: Japanese Patent Laid-Open No. 2000-238161
Patent Publication 4: Japanese Patent Laid-Open No. Hei-7-051315
Patent Publication 5: Japanese Patent Laid-Open No. 2002-325799
Patent Publication 6: Japanese Patent Laid-Open No. 2003-011118
Patent Publication 7: Japanese Patent Laid-Open No. Hei-02-048944

SUMMARY OF THE INVENTION

PROBLEMS TO BE SOLVED BY THE INVENTION

An object of the present invention is to provide a water-absorbent sheet which is capable of accomplishing thinning while avoiding the gel blocking phenomenon, irrespective of having a large content of a water-absorbent resin, while obtaining fundamental properties (fast liquid permeation rate, small amount of re-wet, and small liquid leakage of a water-absorbent sheet, and shape retaining ability of a water-absorbent sheet) as a water-absorbent sheet at a high level, even for a water-absorbent sheet containing a very small amount of pulps.

MEANS TO SOLVE THE PROBLEMS

Specifically, the gist of the present invention relates to:

[1] a water-absorbent sheet comprising a structure in which an absorbent layer containing a water-absorbent resin and an adhesive is sandwiched with nonwoven fabrics from an upper side and a lower side of the absorbent layer, wherein the absorbent layer has a laminate structure that is fractionated into a primary absorbent layer and a secondary absorbent layer with a breathable fractionating layer, and wherein at least one sheet of the nonwoven fabrics is a spunbond nonwoven fabric, and at least the nonwoven fabric at a lower side of the nonwoven fabrics is a hydrophilic nonwoven fabric; and

[2] an absorbent article comprising the water-absorbent sheet as defined in the above [1], sandwiched between a liquid-permeable sheet and a liquid-impermeable sheet.

EFFECTS OF THE INVENTION

The water-absorbent sheet of the present invention exhibits some effects that a water-absorbent sheet is capable of accomplishing avoidance of gel blocking phenomenon and liquid leakage, while obtaining basic properties as a water-absorbent sheet at a high level, by using specified nonwoven fabrics, and further providing a hydrophilic nonwoven fabric as a lower side of a nonwoven fabric of the absorbent layer of a water-absorbent sheet, even for a water-absorbent sheet containing a very small amount of pulps and being thinner than a conventional product.

BRIEF DESCRIPTION OF THE DRAWINGS

[Figure 1] Figure 1 is an enlarged cross-sectional view schematically showing a structure of a water-absorbent sheet of the present invention.

[Figure 2] Figure 2 is an enlarged cross-sectional view schematically showing a structure of another embodiment of a water-absorbent sheet of the present invention.

[Figure 3] Figure 3 is an enlarged cross-sectional view schematically showing a structure of another embodiment of a water-absorbent sheet of the present invention.

[Figure 4] Figure 4 is a schematic view of a measurement apparatus used for measuring an initial water absorption rate and an effective amount of water absorbed of a water-absorbent resin.

[Figure 5] Figure 5 is a schematic view of an apparatus used for carrying out a slope leakage test.

MODES FOR CARRYING OUT THE INTENTION

The water-absorbent sheet of the present invention has one of the features that a water-absorbent sheet comprising a structure in which an absorbent layer containing a water-absorbent resin and an adhesive is sandwiched with nonwoven fabrics from an upper side and a lower side of the absorbent layer, wherein the absorbent layer has a laminate structure that is fractionated into a primary absorbent layer and a secondary absorbent layer with a breathable fractionating layer, and wherein at least one sheet of the nonwoven fabrics is a spunbond nonwoven fabric, and at least a nonwoven fabric at a lower side of the nonwoven fabrics is a hydrophilic nonwoven fabric.

By having the above structure, since appropriate liquid distribution and diffusion are accomplished by a breathable fractionating layer between a primary absorbent layer and a secondary absorbent layer, both the prevention of the gel blocking phenomenon at an early stage of urination and the efficient water permeation into the secondary absorbent layer can be accomplished, and at least a lower side of the nonwoven fabric of the absorbent layer of the water-absorbent sheet has hydrophilicity, whereby rapid liquid permeation to a back side of the water-absorbent sheet is prevented, so that the prevention from liquid leakage can also be accomplished. By having the above structure, especially the liquid slope leakage is dramatically improved. Further, by using a given number of sheets of spunbond nonwoven fabrics having a specified basis weight, a water-absorbent sheet which satisfies all the thinness, shape-retaining ability, and skin feel, and can also be used for an absorbent material for disposable diapers that demand high levels of absorbent properties among the absorbent articles can be provided.

Here, in this water-absorbent sheet, the absorbent layer does not substantially contain a hydrophilic fiber such as pulps which contribute to the immobilization of the water-absorbent resin in the absorbent layer and the shape retention of the absorbent layer, so that the water-absorbent sheet has a very low amount of pulps used.

The number of sheets of the nonwoven fabrics used in sandwiching an absorbent layer having a laminate structure in which the absorbent layers are fractionated by a breathable fractionating layer is not particularly limited. For example, it is preferable that one or more sheets of the nonwoven fabrics are placed on an upper side of the absorbent layer and one or more sheets on a lower side thereof, and it is more preferable that each of the nonwoven fabrics from an upper side and a lower side is one sheet each. In addition, among the nonwoven fabrics used in sandwiching the absorbent layers, at least a nonwoven fabric at a lower side of the absorbent layer is a hydrophilic nonwoven fabric, and more preferably nonwoven fabrics at an upper side and a lower side of the absorbent layer are hydrophilic nonwoven fabrics, from the viewpoint of enhancing absorbent properties such as prevention of liquid slope leakage. The upper side of the absorbent layer of the water-absorbent sheet as used herein refers to a layer closest to a side to which a liquid to be absorbed is supplied at the time of preparing an absorbent article using the water-absorbent sheet, and the lower side of the absorbent layer refers to a furthermost layer away from the side to which a liquid to be absorbed is supplied.

The nonwoven fabrics used in sandwiching the above-mentioned water-absorbent layers are not particularly limited, as long as the nonwoven fabrics are known nonwoven fabrics in the field of art. The nonwoven fabrics include nonwoven fabrics made of polyolefin fibers such as polyethylene (PE) and polypropylene (PP); polyester fibers such as polyethylene terephthalate (PET), polytrimethylene terephthalate (PTT), and polyethylene naphthalate (PEN); polyamide fibers such as nylon; rayon fibers, and other synthetic fibers; nonwoven fabrics produced by mixing cotton, silk, hemp, pulp (cellulose) fibers, or the like, from the viewpoint of liquid permeability, flexibility and strength upon forming into a water-absorbent sheet, and the nonwoven fabrics may be a blend of two or more kinds of fibers.

The nonwoven fabrics differ in optimal synthesis methods, depending upon the materials. A method of producing fibers derived from a chemical synthesis resin such as a polyolefin fiber, a polyester fiber, a polyester fiber, or a polyamide fiber includes a chemical bond method including applying an adhesive resin to a card web to adhere the fibers with each other; a thermal bond method including heating a card web containing low-melting point fibers to dissolve the low-melting point fibers to melt-fuse the fibers with each other; a spunbond method including extruding a thermally molten polymer from nozzles to form a web, and heat-and-pressure fixing the web with a thermal emboss roller (fiber diameter: 10 to 50 $\mu$m); a meltblown method including blowing a hot air to fibers upon extrusion of a thermally molten polymer from nozzles as prescribed in the spunbond method, thereby further thinning the fibers (fiber diameter: less than 10 $\mu$m); and the like.

Among them, the nonwoven fabrics produced according to the chemical bond method and the thermal bond method are more likely to be relatively thick and give a hardened feel. The nonwoven fabrics produced by the meltblown method alone are likely to be weaker in the strength of the nonwoven fabrics and to give a worsened surface feel. The nonwoven fabrics produced by the spunbond method of which fiber diameter is in an appropriate range are more likely to obtain nonwoven fabrics which are relatively thin and give excellent feel.

On the other hand, rayon nonwoven fabrics are synthesized by a spunlace method including intertwining fibers mainly with a water stream, in which a polyester fiber or a thermally fused fiber is properly formulated as occasion demands. The spunlace nonwoven fabrics as described above are in general hydrophilic, and have flexibility.

Among these nonwoven fabrics, at least one sheet of the nonwoven fabrics used in sandwiching the absorbent layers is a spunbond nonwoven fabric. By having the above constitution, both the thinning and the shape-retaining ability of the water-absorbent sheet can be satisfied. It is preferable that as the nonwoven fabrics used in sandwiching the absorbent layers, both the nonwoven fabrics on an upper side and a lower side of an absorbent layer are spunbond nonwoven fabrics. Of a wide variety of spunbond nonwoven fabrics, spunbond nonwoven fabrics made of fibers selected from the group consisting of polyolefin fibers, polyester fibers and blends thereof are more preferred, from the viewpoint of obtaining shape-retaining ability of the water-absorbent sheet, and preventing pass of the water-absorbent resin through the nonwoven fabric. Among the spunbond nonwoven fabrics, multi-layered nonwoven fabrics are even more preferred, among which spunbond-meltblown-spunbond (SMS) nonwoven fabrics and spunbond-meltblown-meltblown-spunbond (SMMS) nonwoven fabrics of polyolefin fibers are more preferably used, and the SMS nonwoven fabrics and the SMMS nonwoven fabrics each made of polypropylene fibers as a main component are especially preferably used. Spunbond nonwoven fabrics of which meltblown parts are optionally added as mentioned above are also encompassed in the spunbond nonwoven fabrics as referred to in the present specification.

Besides, spunlace nonwoven fabrics made of rayon fabrics as a main component are also preferably used as the nonwoven fabrics in the present invention, from the viewpoint of even more enhancing absorbent properties and flexibility upon the formation of the sheet.

At least a lower side of the nonwoven fabrics among the nonwoven fabrics used in sandwiching the absorbent layer is a hydrophilic nonwoven fabric. As the hydrophilic nonwoven fabric, among the nonwoven fabrics mentioned above, those of which materials themselves show hydrophilicity, such as rayon fibers, may be used, or those obtained by subjecting hydrophobic chemical fibers such as polyolefin fibers or polyester fibers to a hydrophilic treatment according to a known method may be used. The method of hydrophilic treatment includes, for example, a method including subjecting, in a spunbond nonwoven fabric, a mixture of a hydrophobic chemical fiber with a hydrophilic treatment agent to a spunbond method to give a nonwoven fabric; a method including carrying a hydrophilic treatment agent along upon the preparation of a spunbond nonwoven fabric with hydrophobic chemical fibers; or a method including obtaining a spunbond nonwoven fabric with a hydrophobic chemical fibers, and thereafter impregnating the nonwoven fabric with a hydrophilic treatment agent, and the like. As the hydrophilic treatment agent, an anionic surfactant such as an aliphatic sulfonic acid salt or a sulfuric acid ester of a higher alcohol; a cationic surfactant such as a quaternary ammonium; a nonionic surfactant such as a polyethylene glycol fatty acid ester, a polyglycerol fatty acid ester, or a sorbitan fatty acid ester; a silicone-based surfactant such as a polyoxyalkylene-modified silicone; and a stain-release agent made of a polyester-based, polyamide-based, acrylic, or urethane-based resin; or the like is used. When the hydrophilicity of the nonwoven fabric is too low, the absorbent properties of the water-absorbent sheet is worsened, and on the other hand, when the hydrophilicity is much higher than a necessary level, the absorbent properties would not be improved to the level equivalent thereto. Therefore, it is desired that the nonwoven fabric has an appropriate level of hydrophilicity. From those viewpoints, the nonwoven fabrics having a degree of hydrophilicity of from 5 to 200 are preferably used, more preferably those having a degree of hydrophilicity of from 8 to 150, even more preferably those having a degree of hydrophilicity of from 10 to 100, and still even more preferably those having a degree of hydrophilicity of from 12 to 80 when measured in accordance with the method for measuring "Degree of Hydrophilicity of Nonwoven Fabric" described later.

It is preferable that the nonwoven fabrics used in sandwiching the absorbent layer have an appropriate basis weight and an appropriate thickness, from the viewpoint of giving flexibility, shape retaining ability and cushioning property to the water-absorbent sheet of the present invention, and speeding up the permeation rate of the water-absorbent sheet. The basis weight thereof is preferably 35 $g/m^2$ or less, more preferably within the range of from 5 to 30 $g/m^2$, even more preferably within the range of from 8 to 25 $g/m^2$, still even more preferably within the range of from 10 to 20 $g/m^2$, and most preferably from 11 to 15 $g/m^2$. In addition, the nonwoven fabrics have a thickness of preferably within the range of from 20 to 800 $\mu$m, more preferably within the range of from 50 to 600 $\mu$m, and even more preferably within the range of from 80 to 450 $\mu$m.

The water-absorbent sheet of the present invention has absorbent layers that are fractionated to two layers, from viewpoint of improving properties of a water-absorbent sheet and achieving stability in quality. The word "fractionated" means, in essence, that a primary absorbent layer and a secondary absorbent layer are individually formed into layers by introduction of a material for fractionating into layers or the like. The structure as described above includes, for example, a laminate structure in which a primary absorbent layer and a secondary absorbent layer are separated by a breathable fractionating layer and bonded; and the like.

The breathable fractionating layer has appropriate breathability and liquid-permeability, which may be a layer in which a particle-form substance such as a water-absorbent resin does not substantially pass therethrough. Specific examples of the preferred materials for the breathable fractionating layer include at least one material selected from the group consisting of the same nonwoven fabrics as those used in sandwiching the above-mentioned absorbent layers, reticular products such as nets having fine pores made of PE or PP fibers; porous films such as perforated films; sanitary papers such as tissue paper; and cellulose-containing synthetic fiber nonwoven fabrics such as air lace nonwoven fabrics made of pulp/PE/PP. The material is preferably the same nonwoven fabric as those used in sandwiching the above-mentioned absorbent layer, and more preferably the above-mentioned spunbond nonwoven fabric, from the viewpoint of absorbent properties (permeation rates, slope leakage, and the like) and the like of the water-absorbent sheet.

The thickness and the basis weight of the breathable fractionating layer are not particularly limited. Exemplifying more preferred embodiments, the breathable fractionating layer has a basis weight of preferably 35 $g/m^2$ or less, more preferably in the range of from 5 to 30 $g/m^2$, even more preferably in the range of from 8 to 25 $g/m^2$, still even more preferably in the range of from 10 to 20 $g/m^2$, and most preferably in the range of from 11 to 15 $g/m^2$. The breathable fractionating layer has a thickness preferably in the range of from 20 to 800 $\mu$m, more preferably in the range of from 50 to 600 $\mu$m, and even more preferably in the range of from 80 to 450 $\mu$m. It is preferable that the breathable fractionating layer is a nonwoven fabric having an appropriate basis weight and an appropriate thickness, from the viewpoint of giving the water-absorbent sheet of the present invention flexibility, shape retaining ability and cushioning property, and speeding up the permeation rate of the water-absorbent sheet and preventing slope leakage of a liquid.

As the kinds of the water-absorbent resins, commercially available water-absorbent resins can be used. For example, the water absorbent resin includes hydrolysates of starch-acrylonitrile graft copolymers, neutralized products of starch-acrylic acid graft polymers, saponified products of vinyl acetate-acrylic acid ester copolymers, partially neutralized products of polyacrylic acid, and the like. Among them, the partially neutralized products of polyacrylic acids are preferred, from the viewpoint of production amount, production costs, water-absorbent properties, and the like. Methods for synthesizing partially neutralized products of polyacrylic acid include reversed phase suspension polymerization method and aqueous solution polymerization method. Among them, the water-absorbent resins obtained according to reversed phase suspension polymerization method are more preferably used, from the viewpoint of excellent flowability of the resulting particles, smaller amounts of fine powder, high water-absorbent properties, such as water absorption capacity and water-absorption rate.

More specifically, an embodiment where a water-absorbent resin used in at least one of the absorbent layers is a water-absorbent resin obtained by reversed phase suspension polymerization method is preferred, an embodiment where a water-absorbent resin used in a secondary absorbent layer is a water-absorbent resin obtained by reversed phase suspension polymerization method is more preferred, and an embodiment where both the water-absorbent resins used in the primary absorbent layer and the secondary absorbent layer are water-absorbent resins obtained by reversed phase suspension polymerization method is even more preferred.

The partially neutralized product of a polyacrylic acid has a degree of neutralization of preferably 50% by mol or more, and even more preferably from 70 to 90% by mol, from the viewpoint of increasing an osmotic pressure of the water-absorbent resin, thereby increasing water absorbent properties.

The water-absorbent resin is contained in the water-absorbent sheet, in a total amount of a primary absorbent layer and a secondary absorbent layer, in the water-absorbent sheet of preferably from 100 to 1000 g per 1 m$^2$, i.e. 100 to 1000 g/m$^2$, more preferably from 150 to 800 g/m$^2$, even more preferably from 200 to 700 g/m$^2$, and still even more preferably from 220 to 600 g/m$^2$, from the viewpoint of obtaining sufficient water-absorbent properties even when a water-absorbent sheet of the present invention is used for an absorbent article. The water-absorbent resin is contained in an amount of preferably 100 g/m$^2$ or more, from the viewpoint of exhibiting sufficient water-absorbent properties as a water-absorbent sheet, thereby suppressing re-wetting, and the water-absorbent resin is contained in a total amount of preferably 1000 g/m$^2$ or less, from the viewpoint of suppressing the gel blocking phenomenon from being caused, exhibiting liquid diffusibility as a water-absorbent sheet, and further improving a liquid permeation rate.

The resin ratio (mass ratio) of the primary absorbent layer/secondary absorbent layer is preferably within the range of from primary absorbent layer/secondary absorbent layer = 98/2 to 50/50, more preferably with the range of from primary absorbent layer/secondary absorbent layer = 98/2 to 60/40, even more preferably within the range of from primary absorbent layer/secondary absorbent layer = 95/5 to 70/30, and still even more preferably within the range of from primary absorbent layer/secondary absorbent layer = 95/5 to 80/20. The ratio of the primary absorbent layer is preferably 98 or less, from the viewpoint of sufficiently exhibiting water-absorbent properties of a secondary absorbent layer, thereby preventing liquid leakage, and the ratio of the primary absorbent layer is preferably in a ratio of 50 or more, from the viewpoint of increasing dry feel of the primary absorbent layer and lowering the amount of re-wet after liquid absorption.

The absorbent properties of the water-absorbent sheet of the present invention are influenced by the water-absorbent properties of the water-absorbent resin used. Therefore, it is preferable that the water-absorbent resin to be used in the present invention is those selected with favorable ranges in water absorbent properties such as absorption capacity (expressed by indices such as effective amount of water absorbed and water-retention capacity), and water absorption rate of the water-absorbent resin, by taking the constitution of each component of the water-absorbent sheet or the like into consideration. Therefore, the kinds of the water-absorbent resin of the primary absorbent layer and the kinds of the water-absorbent resin of the secondary absorbent layer may be identical to or different from each other.

In the present specification, the water-retention capacity of the water-absorbent resin is evaluated as a water-retention capacity of saline solution. The water-absorbent resin has a water-retention capacity of saline solution of preferably 25 g/g or more, more preferably from 25 to 60 g/g, and even more preferably from 30 to 50 g/g, from the viewpoint of absorbing a liquid in a larger amount, and preventing the gel blocking phenomenon while keeping the gel strong during absorption. The water-retention capacity of saline solution of the water-absorbent resin is a value obtainable by a measurement method described in Examples set forth below.

In the present specification, the water absorption rate of the water-absorbent resin is evaluated as a water absorption rate of saline solution. The water-absorbent resin has a water-absorption rate of saline solution of preferably from 1 to 70 s, more preferably from 2 to 60 s, and even more preferably from 3 to 55 s, from the viewpoint of speeding up the permeation rate of the water-absorbent sheet of the present invention, thereby preventing a liquid leakage upon use in an absorbent article. The water-absorption rate of the water-absorbent resin as used herein is a value obtainable by a measurement method described in Examples set forth below.

In the water-absorbent sheet of the present invention, it is preferable that there is a positive difference in values between the water absorption rate of saline solution of a water-absorbent resin used in the primary absorbent layer and the rate of that used in the secondary absorbent layer. The greater the difference therebetween, effects of avoiding the stagnation of a liquid in the primary absorbent layer, to thereby increase dry feel, and effects of preventing a liquid leakage are even more strongly exhibited. Specifically, (the water absorption rate of saline solution of a water-absorbent resin used in the primary absorbent layer) - (the water absorption rate of saline solution of a water-absorbent resin used in the secondary absorbent layer) is preferably 10 seconds or more, more preferably 15 seconds or more, and even more preferably 20 seconds or more.

From the viewpoint of improving absorbent properties of the water-absorbent sheet, in a still even more preferred embodiment, in a case where the kind of the water-absorbent resin of the primary absorbent layer and the kind of the water-absorbent resin of the secondary absorbent layer are different, the water-absorbent resin used in the primary absorbent layer has a water-absorption rate of saline solution of preferably from 20 to 70 s, more preferably from 25 to 60 s, and even more preferably from 30 to 55 s, from the viewpoint of speeding up the permeation rate of the water-absorbent sheet of the present invention, thereby avoiding residence of a liquid in the primary absorbent layer, to increase dry feel to the skin upon use in an absorbent article. On the other hand, the water-absorbent resin used in the secondary absorbent layer has a water-absorption rate of saline solution of preferably from 1 to 40 s, more preferably from 2 to 30 s, even more preferably from 2 to 20 s and still even more preferably from 3 to 15 s, from the viewpoint of reducing leakage at slope of a water-absorbent sheet of the present invention, thereby preventing unpleasant feel caused by liquid leakage upon use in an absorbent article.

The median particle size of the water-absorbent resin used in the present invention is not particularly limited, and the water-absorbent resin has a median particle size of preferably from 100 to 600 μm, more preferably from 150 to 550 μm, and even more preferably from 200 to 500 μm. It is preferable that the water-absorbent resin has a median particle size of 100 $\mu$m or more, from the viewpoint of avoiding the use of fine powder which has a worsened flowability as a powder and is likely to cause the gel blocking phenomenon upon water absorption, thereby increasing operability workability during the production of a water-absorbent sheet and basic properties of a water-absorbent sheet. It is preferable that the water-absorbent resin has a median particle size of 600 $\mu$m or less, from the viewpoint of reducing the rugged feel of the water-absorbent sheet, thereby improving feel.

In general, those water-absorbent resins having a large median particle size are likely to have slower water absorption rate, and those having a small median particle size are likely to have a faster water absorption rate. It is possible that a resin having a water absorption rate that is fast in the same level as that used in a secondary absorbent layer in the present invention is obtained, for example, when a median particle size in a conventional water-absorbent resin is made to a size of less than 100 $\mu$m. However, when a median particle size of a water-absorbent resin is made small to a size of less than 100 $\mu$m, flowability as powder become greatly worsened, so that there arise some problems such as worsening of working environment due to powdered state, and lowering in productivity due to scattering and detachment of a water-absorbent resin from a nonwoven fabric. Further, when the amount of fine powder of a water-absorbent resin increases, the gel block phenomenon as mentioned above is more likely to take place, thereby leading to lowering of the absorbent properties of the water-absorbent sheet. Besides, an adhesive effect is lowered probably because a water-absorbent resin having a small median particle size is more likely to cover over an adhesive, so that the adhesive effect is lowered, thereby making the laminate more likely to be exfoliated.

In order to avoid these problems, it is preferable to use a water-absorbent resin having an appropriate median particle size and a fast water absorption rate in a secondary absorbent layer. In order to obtain a water-absorbent resin as described above, it is preferable that a specified method for producing a water-absorbent resin is used, for example, it is preferable to use an aqueous solution polymerization method in which continuous bubbles are introduced by foaming during polymerization, or to use a reversed phase polymerization method using a specified emulsifying agent, among which the latter method is more preferred, from the viewpoint of having high water absorbent properties and stably obtaining a fast water absorption rate. As a specified emulsifying agent, a nonionic surfactant having an appropriate hydrophilicity is preferably used, and a water-absorbent resin from a reversed phase suspension polymerization using them is usually obtained in a spherical or granular form, and an agglomerated form thereof. The resin having the form mentioned above is preferably used from the viewpoint that not only pulverization is hardly needed, but also a water-absorbent sheet has excellent flowability as a powder, and has excellent operability during the production, or the like.

On the other hand, it is preferable that the water-absorbent resin used in the primary absorbent layer has, in addition to the water absorption rate of saline solution within the range mentioned above, a specified initial water absorption rate and a specified effective amount of water absorbed. The initial water absorption rate is expressed as an amount of water absorbed of a liquid per second in the water absorption period of from 0 to 30 s, and the initial water absorption rate is preferably 0.35 mL/s or less, from the viewpoint of suppressing the gel blocking phenomenon from being caused at an initial stage of the liquid permeation, thereby accelerating liquid diffusion in a primary absorbent layer, and efficiently transmitting the liquid into a secondary absorbent layer. The initial water absorption rate is more preferably from 0.05 to 0.30 mL/s, and even more preferably from 0.10 to 0.25 mL/s. The initial water absorption rate is more preferably 0.05 mL/s or more, from the viewpoint of obtaining dry feel to skin in an initial stage of liquid permeation while diffusing the liquid.

The effective amount of water absorbed of the water-absorbent resin used in the primary absorbent layer, in terms of an effective amount of water absorbed for saline solution, is preferably 45 mL/g or more, more preferably from 50 to 80 mL/g, and even more preferably from 55 to 70 mL/g. The water-absorbent resin has an effective amount of water absorbed of preferably 45 mL/g or more, from the viewpoint of allowing a water-absorbent resin to absorb more liquid, and reducing the amount of re-wet, thereby obtaining dry feel, and the water-absorbent resin has an effective amount of water absorbed of preferably 80 mL/g or less, from the viewpoint of providing appropriate crosslinking of the water-absorbent resin, thereby keeping the gel strong upon absorption and preventing gel blocking.

As mentioned above, the water absorption rate of the water-absorbent resin is likely to be slowed if a median particle size becomes large. However, with regard to the initial water absorption rate (mL/s), this effect is small even when a median particle size is made large in a conventional water-absorbent resin, so that in a conventional water-absorbent resin, even if a median particle size is made to a size of, for example, 600 $\mu$m or more, an initial water absorption rate needed in the present invention is less likely to be obtained. Moreover, if a median particle size of a water-absorbent resin is made as large as 600 $\mu$m or more, it is undesirable because feel in the water-absorbent sheet is likely to be worsened. In view of the above, in a case where a water-absorbent resin having a particular range of particle sizes is used, as a method of controlling an initial water absorption rate of a primary absorbent layer to a particular range, for example, a method for producing a water-absorbent resin comprising increasing a crosslinking density of a water-absorbent resin with a crosslinking agent, or homogeneously coating a surface of a water-absorbent resin with a hydrophobic additive, or carrying out reversed phase suspension polymerization using a specified emulsifying agent, or the like is considered.

However, if a crosslinking density of a water-absorbent resin is increased with a crosslinking agent, a specified initial water absorption rate might be satisfied but at the same time an effective amount of water absorbed (absorption capacity) of the water-absorbent resin is lowered, so that it is difficult to obtain a water-absorbent resin satisfying both a specified initial water absorption rate and an effective amount of water absorbed.

Accordingly, in the present invention, a water-absorbent resin used in a primary absorbent layer is more preferably those in which a hydrophobic additive is homogeneously coated on a surface of a water-absorbent resin, and those produced according to reversed phase suspension polymerization using a specified emulsifying agent, from the viewpoint of facilitation in the production of a water-absorbent resin having both a specified initial water absorption rate and a specified effective amount of water absorbed, among which the latter method is even more preferred from the viewpoint of high water-absorbent properties. As a specified emulsifying agent, a nonionic surfactant having an appropriate hydrophobicity is preferably used, and a water-absorbent resin from a reversed phase suspension polymerization using them is obtained usually in a spherical or American football-shaped form, or an agglomerated form thereof. The resin having the above form is preferably used from the viewpoint that pulverization is hardly needed, and has excellent flowability as powder and excellent operability during the production of a water-absorbent sheet.

Here, the effective amount of water absorbed of the water-absorbent resin used in a secondary absorbent layer is not particularly limited, and the effective amount of water absorbed is preferably 30 mL/g or more, and even more preferably 45 mL/g or more.

The effective amount of water absorbed of the water-absorbent resin as used herein is a value obtainable by a measurement method described in Examples set forth below.

The adhesive contained in the absorbent layer includes, for example, rubber-based adhesives such as natural rubbers, butyl rubbers, and polyisoprene; styrene-based elastomer adhesives such as styrene-isoprene block copolymers (SIS), styrene-butadiene block copolymers (SBS), styrene-isobutylene block copolymers (SIBS), and styrene-ethylenebutylene-styrene block copolymers (SEBS); ethylene-vinyl acetate copolymer (EVA) adhesives; ethylene-acrylic acid derivative copolymer-based adhesives such as ethylene-ethyl acrylate copolymer (EEA), and ethylene-butyl acrylate copolymer (EBA); ethylene-acrylic acid copolymer (EAA) adhesives; polyamide-based adhesives such as copolymer nylons and dimer acids polyamides; polyolefin-based adhesives such as polyethylenes, polypropylenes, atactic polypropylenes, and copolymeric polyolefins; polyester-based adhesives such as polyethylene terephthalate (PET), polybutylene terephthalate (PBT), and copolymeric polyesters; and acrylic-based adhesives, and these adhesives may be used together in two or more kinds. In the present invention, the ethylene-vinyl acetate copolymer adhesives, the styrene-based elastomer adhesives, the polyolefin-based adhesives, and the polyester-based adhesives are preferred, from the viewpoint of high adhesive strength, thereby making it possible to prevent exfoliation of a nonwoven fabric and scattering of the water-absorbent resin in the water-absorbent sheet.

The adhesive has a melting temperature of preferably from 60° to 180°C, more preferably from 70° to 150°C, and even more preferably from 75° to 125°C, from the viewpoint of sufficiently fixing a water-absorbent resin to a nonwoven fabric, and at the same time preventing thermal deterioration or deformation of the nonwoven fabric. Here, in the water-absorbent sheet of the present invention, in the process of producing a water-absorbent sheet, after melting, the adhesive is adhered to a nonwoven fabric or a hydrophilic resin in a solid state by cooling the molten adhesive.

The adhesive in the water-absorbent sheet is contained in an amount preferably in the range of from 0.05 to 2.0 times, more preferably in the range of from 0.08 to 1.5 times, and even more preferably in the range of from 0.1 to 1.0 time the amount of the water-absorbent resin contained (mass basis). It is preferable that the adhesive is contained in an amount of 0.05 times or more, from the viewpoint of having sufficient adhesion, thereby preventing exfoliation of the nonwoven fabrics themselves or scattering of the water-absorbent resin, and increasing shape retaining ability of a water-absorbent sheet. It is preferable that the adhesive is contained in an amount of 2.0 times or less, from the viewpoint of avoiding the inhibition of the swelling of the water-absorbent resin due to too strong adhesion to each other, thereby improving a permeation rate or liquid leakage of a water-absorbent sheet.

The absorbent layer contains a water-absorbent resin and an adhesive, and the absorbent layer is formed by, for example, evenly dispersing a mixed powder of a water-absorbent resin and an adhesive on a nonwoven fabric, and further overlaying with a breathable fractionating layer, and subjecting overlaid layers to heating, if necessary, heating under pressure near a melting temperature of the adhesive. Alternatively, the absorbent layer is formed by applying an adhesive to a nonwoven fabric, thereafter evenly dispersing a water-absorbent resin, overlaying with a breathable fractionating layer, and subjecting overlaid layers to heating, if necessary, under pressure.

The water-absorbent sheet of the present invention can be produced by a method, for example, as described in a method as described hereinbelow, utilizing a conventional method.

(a) A mixed powder of a water-absorbent resin and an adhesive is evenly dispersed over a nonwoven fabric, a breathable fractionating layer is further overlaid thereto, and the overlaid layers are together subjected to heat-and-pressure fixing near a melting temperature of an adhesive. The mixed powder was dispersed over the breathable fractionating layer in the same manner as above, and a nonwoven fabric is again subjected to heat-and-pressure fixing.

(b) A mixed powder of a water-absorbent resin and an adhesive is evenly dispersed over a nonwoven fabric, a breathable fractionating layer is further overlaid thereto, thereafter the mixed powder is again dispersed over the breathable fractionating layer, a nonwoven fabric is overlaid thereto, and the overlaid layers are together subjected to heat-and-pressure fixing.

(c) A mixed powder of a water-absorbent resin and an adhesive is evenly dispersed over a nonwoven fabric, and passed through a heating furnace to fix the powder to an extent that the powder does not scatter. A breathable fractionating layer is further overlaid thereto, thereafter the mixed powder is again dispersed over the breathable fractionating layer, a nonwoven fabric is overlaid thereto, and the overlaid layers are together subjected to heat-and-pressure fixing.

(d) An adhesive is melt-coated over a nonwoven fabric, a water-absorbent resin is then evenly dispersed thereto to form a layer, and further a breathable fractionating layer to which an adhesive is melt-coated is overlaid from an upper side in a manner that a coated side of the adhesive is facing the side of the dispersed water-absorbent resin, the overlaid layers are subjected to pressing, or pressing, if necessary, with heating, using a roller press. Layers of an adhesive and a water-absorbent resin are further formed over the breathable fractionating layer, and the same procedures as above are carried out by changing the above-mentioned breathable fractionating layer to a nonwoven fabric.

A laminate structure in which has a structure that an absorbent layer containing a water-absorbent resin and an adhesive is sandwiched with nonwoven fabrics from an upper side and a lower side of the absorbent layer, and that the absorbent layers are fractionated into a primary absorbent layer and a secondary absorbent layer can be accomplished by, for example, producing a water-absorbent sheet according to the method shown in any one of these (a) to (d). Among them, the methods of (b) and (d) are more preferred, from the viewpoint of convenience in the production method and high production efficiency.

Here, among the methods exemplified in (a) to (d), the water-absorbent sheets can be produced by separately selecting methods of adhering a primary absorbent layer and a secondary absorbent layer, to be used in combination. The water-absorbent sheet may be subjected to emboss treatment during heat-and-pressure fixing in the production of a sheet or after the production of the sheet, for the purposes of improving the feel and improving strength of the water-absorbent sheet.

In addition, the water-absorbent sheet of the present invention may properly be formulated with an additive such as a deodorant, an antibacterial agent, or a gel stabilizer.

The water-absorbent sheet of the present invention has one feature in the viewpoint of enabling thinning of the sheet. When the use in absorbent articles is taken into consideration, the water-absorbent sheet has a thickness, on a dry basis, of preferably 3 mm or less, more preferably 2.5 mm or less, even more preferably from 0.5 to 2 mm, and still even more preferably from 0.8 to 1.6 mm.

Further, the water-absorbent sheet of the present invention has one feature in that a liquid has a fast permeation rate, and the water-absorbent sheet has a total permeation rate of preferably 100 seconds or less, more preferably 90 seconds or less, and even more preferably 80 seconds or less, when the use as an absorbent article is taken into consideration.

Further, the water-absorbent sheet of the present invention has one feature in that a liquid has smaller liquid leakage, and the water-absorbent sheet has a leakage index of preferably 100 or less, more preferably 50 or less, and even more preferably 30 or less, when the use as an absorbent article is taken into consideration.

Further, the water-absorbent sheet of the present invention has one feature in the aspect that shape retaining ability of the water-absorbent sheet is high. In the production process of an absorbent article, when a water-absorbent sheet is stretched in the machine feeding direction (longitudinal direction), a so-called neck-down takes place, which is a phenomenon in which the shape of the water-absorbent sheet is lost, and the width at an intersecting direction becomes narrow. In a case where the stretched water-absorbent sheet is cut into a give size during the steps, but does not recover the original state from the neck down, even after stretching strength is removed, variances of the lengths in the intersecting direction are caused, thereby giving disadvantageous influences to the quality stability of the absorbent article. The smaller the neck down of such a water-absorbent sheet, the more favorable.

The shape retaining ability of the water-absorbent sheet is measured by an anti-neck down percentage described later, and the water-absorbent sheet has an anti-neck down percentage of preferably 85% or more, more preferably 90% or more, and even more preferably 95% or more, in consideration of its use as an absorbent article.

A water-absorbent sheet satisfying all the properties as mentioned above is very highly preferable in consideration of its use as an absorbent article.

Further, since the water-absorbent sheet of the present invention has a very small amount of a material derived from nature, such as crushed pulp, consideration has been made to the environment while having high performance in thickness, permeation rate, and a leakage index as mentioned above. The proportion of the natural material is preferably 20% or less, more preferably 10% or less, even more preferably 5% or less, and still even more preferably 3% or less. The proportion of the natural material is calculated by dividing a total content of pulp, cotton and the like contained in very small amounts as the constituents of the water-absorbent sheet by mass of the water-absorbent sheet.

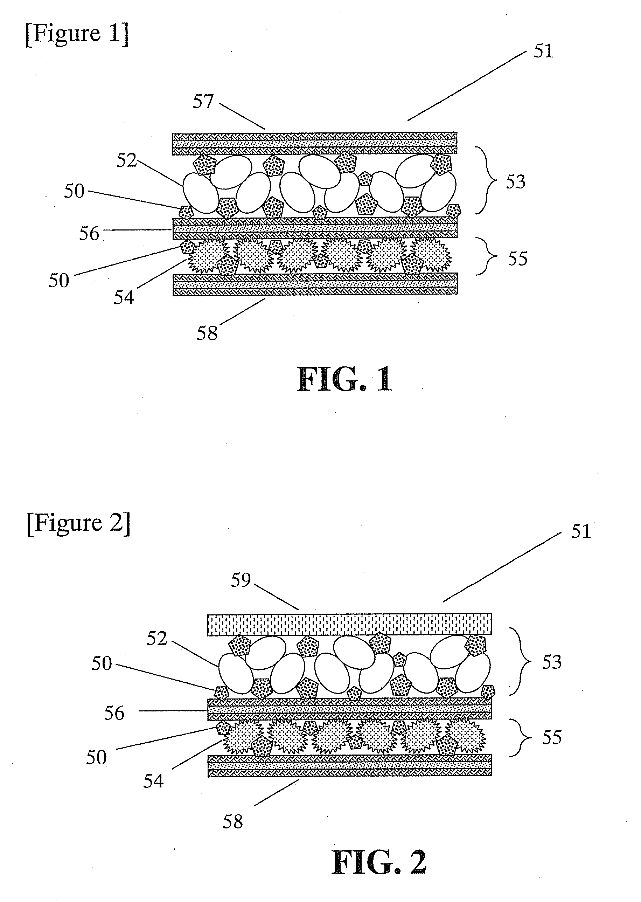

Next, the structure of the water-absorbent sheet of the present invention will be explained by referring to Figure 1. Here, Figure 1 is an enlarged cross-sectional view schematically showing a structure of a water-absorbent sheet of the present invention.

A water-absorbent sheet 51 shown in Figure 1 comprises a primary absorbent layer 53 containing a first water-absorbent resin 52 and an adhesive 50, and a secondary absorbent layer 55 containing a second water-absorbent resin 54 and an adhesive 50. Here, the primary absorbent layer refers to a side to which a liquid to be absorbed is supplied upon the preparation of an absorbent article using the water-absorbent sheet, and the secondary absorbent layer refers to a side to which a liquid to be absorbed is supplied via the primary absorbent layer. Therefore, the water-absorbent sheet of the present invention has a structure in which a primary absorbent layer 53 and a secondary absorbent layer 55 are indirectly laminated via a breathable fractionating layer 56 in a thickness direction of a water-absorbent sheet 51, as shown in Figure 1.

Moreover, in Figure 1, the absorbent layer is fractionated into a primary absorbent layer and a secondary absorbent layer, so that the two absorbent layers do not substantially mix with each other. A water-absorbent sheet 51 in Figure 1 has a five-layer structure, comprising a primary absorbent layer 53, a secondary absorbent layer 55, a breathable fractionating layer 56 and a nonwoven fabric 57 positioned on an outer side of the primary absorbent layer, and a hydrophilic nonwoven fabric 58 positioned at an outer surface of the secondary absorbent layer 55.

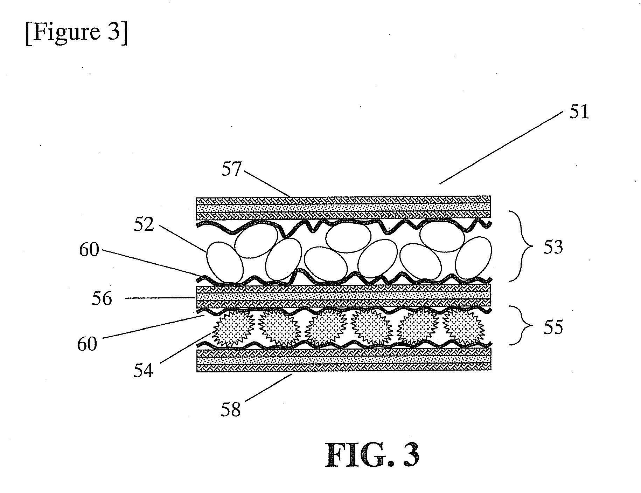

Incidentally, the water-absorbent sheets shown in Figures 2 and 3 are also exemplifications of other embodiments of the water-absorbent sheet of the present invention. Figure 2 is an example where other nonwoven fabric 59 is used in place of the nonwoven fabric 57 in Figure 1. Figure 3 is an example where an adhesive 60 is melt-coated over a hydrophilic nonwoven fabric 58 and the like.

In a preferred embodiment of the water-absorbent sheet of the present invention, a nonwoven fabric at an upper side of an absorbent layer of the water-absorbent sheet is a hydrophilic nonwoven fabric, without being particularly limited to in its kinds, and a nonwoven fabric at a lower side of the absorbent layer of the water-absorbent sheet is a hydrophilic nonwoven fabric, and preferably a spunbond nonwoven fabric, and the breathable fractionating layer is a hydrophilic nonwoven fabric, preferably a spunbond nonwoven fabric.

The absorbent article of the present invention has a structure in which a water-absorbent sheet of the present invention is sandwiched between a liquid-permeable sheet and a liquid-impermeable sheet. The absorbent article includes, for example, disposable diapers, incontinence pads, sanitary napkins, pet sheets, drip sheets for foods, water blocking materials for electric power cables, and the like. Further, as the liquid-permeable sheet and the liquid-impermeable sheet, known ones in the technical field of the absorbent articles can be used without particular limitations. The absorbent article can be produced by a known method.

<u>EXAMPLES</u>

The present invention will be specifically described hereinbelow by the Examples, without intending to limit the scope of the present invention thereto.

The properties of the water-absorbent resin and the water-absorbent sheet were measured in accordance with the following methods.

< Water-Retention Capacity of Saline Solution of Water-Absorbent Resin >

The amount 2.0 g of water-absorbent resin was weighed in a cotton bag (Cottonbroad No. 60, width 100 mm x length 200 mm), and placed in a 500 mL-beaker. Saline solution (0.9% by mass aqueous solution of sodium chloride, hereinafter referred to the same) was poured into the cotton bag in an amount of 500 g at one time, and the saline solution was dispersed so as not to cause an unswollen lump of the water-absorbent resin. The upper part of the cotton bag was tied up with a rubber band, and the cotton bag was allowed to stand for 1 hour, to sufficiently make the water-absorbent resin swollen. The cotton bag was dehydrated for 1 minute with a dehydrator (manufactured by Kokusan Enshinki Co., Ltd., product number: H-122) set to have a centrifugal force of 167G. The mass Wa (g) of the cotton bag containing swollen gels after the dehydration was measured. The same procedures were carried out without adding water-absorbent resin, and the empty mass Wb (g) of the cotton bag upon wetting was measured. The water-retention capacity of saline solution of the water-absorbent resin was calculated from the following formula.

$$\text{Water-Retention Capacity of Saline Solution (g/g) of}$$

$$\text{Water-Absorbent Resin}$$

$$= [\text{Wa - Wb}] \text{ (g)/Mass (g) of Water-Absorbent Resin}$$

< Initial Water Absorption Rate and Effective Amount of Water Absorbed of Water-Absorbent Resin >

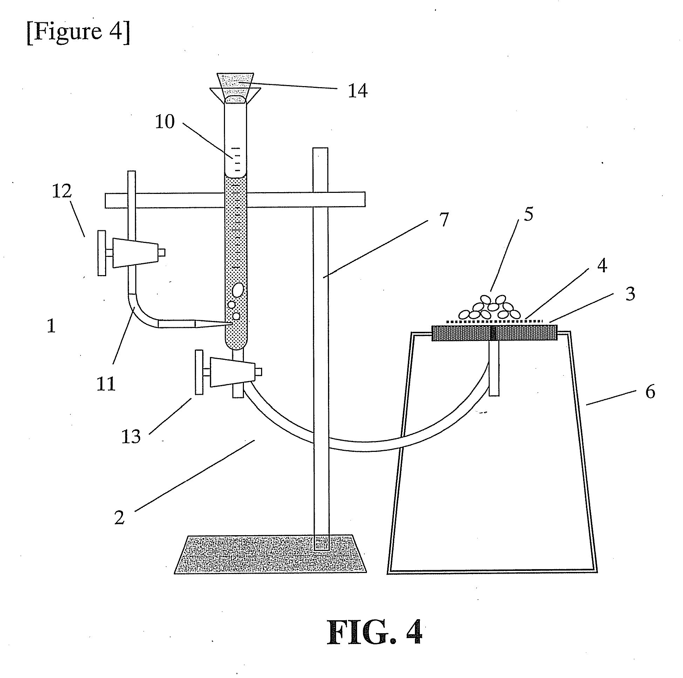

The initial water absorption rate and the effective amount of water absorbed of the water-absorbent resin were measured using a measurement apparatus as shown in Figure 4.
The measurement apparatus comprised a burette section 1, a lead tube 2, a measuring platform 3, a nonwoven fabric 4, a stand 6, and a clamp 7. The burette section 1 was connected to a rubber plug 14 at the top of a burette 10 which had been graduated in units of 0.1 mL, and an air inlet tube 11 and a cock 12 at the bottom portion thereof, and further, the burette 10 had a cock 13 at a tip end of bottom portion thereof. The burette section 1 was fixed with a clamp 7. The lead tube 2 was attached between the burette section 1 and the measuring platform 3. The lead tube 2 had an inner diameter of 6 mm. A hole of a diameter of 2 mm is made at the central section of the measuring platform 3, and the lead tube 2 is connected thereto. The measuring platform 3 was supported at an appropriate height by a stand 6.
The measurements of the initial water absorption rate and the effective amount of water absorbed using the measurement apparatus as described above were carried out by the following procedures. The measurements were taken indoors at a temperature of 25°C and humidity of from 45 to 75%. First, the cock 12 and the cock 13 at the burette section 1 were closed, and a 0.9% by mass aqueous sodium chloride solution adjusted to 25°C was poured from the top of the burette 10 and the top of the burette was plugged with the rubber plug 14. Thereafter, the cock 12 and the cock 13 at the burette section 1 were opened. Next, an internal of a lead tube 2 was filled with a 0.9% by mass aqueous sodium chloride solution while removing bubbles, and the height of the measuring platform 3 was adjusted so that a water level of a 0.9% by mass aqueous sodium chloride solution coming out of a lead tube inlet at the central portion of the measuring platform 3 and an upper side of the measuring platform 3 would be at the same height.
Next, a nonwoven fabric 4 cut into dimensions of 30 mm x 30 mm (hydrophilic rayon spunlace having a basis weight of 25 $g/m^2$) was spread on a lead tube inlet at the central portion of the measuring platform 3, and the nonwoven fabric was allowed to absorb water until reaching an equilibrium. In the state where a nonwoven fabric absorbed water, the generation of bubbles from an air lead tube 11 to a burette 10 was observed, and having confirmed that the generation of bubbles stopped within several minutes, it was judged that an equilibrium was reached. After equilibration, the scales of the burette 10 were read off to confirm a zero point.
Separately, 0.10 g of a water-absorbent resin 5 was measured accurately, and supplied at one time to a central part of a nonwoven fabric 4. An amount of a 0.9% by mass aqueous sodium chloride solution reduced inside the burette 10 (in other words, an amount of a 0.9% by mass aqueous sodium chloride solution absorbed by the particles of a water-absorbent resin 5) was sequentially read off, and a reduced portion of a 0.9% by mass aqueous sodium chloride solution after 30 seconds counted from the supplying of a water-absorbent resin 5 Wc (mL) was recorded as an amount of water absorbed per 0.10 g of a water-absorbent resin. Incidentally, the measurement of the reduced portion was continued to be measured even after the passage of 30 s, and a reduced portion of a 0.9% by mass aqueous sodium chloride solution after 30 minutes counted from the supplying of a water-absorbent resin 5 Wd (mL) was measured, to complete the measurement. The measurements were taken 5 times per one kind of a water-absorbent resin, and a 3-point average excluding a minimum value and a maximum value was used.
The amount of a 0.9% by mass aqueous sodium chloride solution absorbed to a water-absorbent resin 5 after 30 s from the supply Wc (mL) was converted to an amount of water absorbed per 1 g of a water-absorbent resin, and a quotient obtained by further dividing the resulting converted value by 30 (s) was defined as an initial water absorption rate (mL/s) of the water-absorbent resin. In other words, the initial water absorption rate (mL/s) =Wc ÷ (0.10 x 30).
In addition, an amount of a 0.9% by mass aqueous sodium chloride solution absorbed after the passage of 30 minutes from the supply of a water-absorbent resin 5 Wd (mL) was converted to an amount of water absorbed per 1 g of a water-absorbent resin, and defined as an effective amount of water absorbed (mL/g) of saline solution of the water-absorbent resin. In other words, the effective amount of water absorbed (mL/g) = Wd ÷ 0.10.

< Water Absorption Rate of Saline Solution of Water-Absorbent Resin >

This test was conducted in a room temperature-controlled to 25° $\pm$ 1°C. The amount 50 $\pm$ 0.1 g of physiological saline solution was weighed out in a 100 mL beaker, and a magnetic stirrer bar (8 mm$\phi$ x 30 mm, without a ring) was placed therein. The beaker was immersed in a thermostat, of which liquid temperature was controlled to 25° $\pm$ 0.2°C. Next, the beaker was placed over the magnetic stirrer so that a vortex was generated in physiological saline solution at a rotational speed of 600 r/min, the water-absorbent resin was then quickly added in an amount of 2.0 $\pm$ 0.002 g to the above beaker, and the time period (seconds) from a point of addition of the water-absorbent resin to a point of convergence of the vortex of the liquid surface was measured with a stopwatch, which was defined as a water absorption rate of the saline solution of the water-absorbent resin.

< Median Particle Size of Water-Absorbent Resin >

Unless specified otherwise, the particle size of the water-absorbent resin is defined as a median particle size, and measured as follows. An amorphous silica (Sipernat 200, Degussa Japan) was mixed in an amount of 0.5 g as a lubricant with 100 g of a water-absorbent resin.
The above-mentioned water-absorbent resin was allowed to pass though a JIS standard sieve having a sieve opening of 250 $\mu$m, and a median particle size was measured using a combination of sieves of (A) in a case where the resin is allowed to pass in an amount of 50% by mass or more, or a combination of sieves of (B) in a case where 50% by mass or more of the resin remains on the sieve.
(A) JIS standard sieves, a sieve having an opening of 425 $\mu$m, a sieve having an opening of 250 $\mu$m, a sieve having an opening of 180 $\mu$m, a sieve having an opening of 150 $\mu$m, a sieve having an opening of 106 $\mu$m, a sieve having an opening of 75 $\mu$m, a sieve having an opening of 45 $\mu$m, and a receiving tray were combined in order from the top.
(B) JIS standard sieves, a sieve having an opening of 850 $\mu$m, a sieve having an opening of 600 $\mu$m, a sieve having an opening of 500 $\mu$m, a sieve having an opening of 425 $\mu$m, a sieve having an opening of 300 $\mu$m, a sieve having an opening of 250 $\mu$m, a sieve having an opening of 150 $\mu$m, and a receiving tray were combined in order from the top.
The above-mentioned water-absorbent resin was placed on an uppermost sieve of the combined sieves, and shaken for 20 minutes with a rotating and tapping shaker machine to classify the resin.
After classification, the relationships between the opening of the sieve and an integral of a mass percentage of the water-absorbent resin remaining on the sieve were plotted on a logarithmic probability paper by calculating the mass of the water-absorbent resin remaining on each sieve as a mass percentage to an entire amount, and accumulating the mass percentages in order, starting from those having larger particle diameters. A particle diameter corresponding to a 50% by mass cumulative mass percentage is defined as a median particle size by joining the plots on the probability paper in a straight line.

< Measurement of Thickness of Water-Absorbent Sheet >

The thickness of the resulting water-absorbent sheet was measured using a thickness measurement instrument (manufactured by Kabushiki Kaisha Ozaki Seisakusho, model number: J-B). As the measurement sites, three sites were arbitrarily determined in a longitudinal direction, on the left end, the center, and the right end; for example, in a water-absorbent sheet of 10 cm x 30 cm, the left end was set at a site 3 cm away from the left side, the center was set at a site 15 cm away therefrom, and the right end was set at a site 27 cm away therefrom. As the width direction, a uniform central part was measured. The measurement value for thickness was obtained by measuring three times at each site and averaging the values for each site. Further, the values at the left end, the center, and the right end were averaged, to give a thickness of an overall water-absorbent sheet.

< Shape Retaining Ability (Anti-Neck Down Percentage) of Water-Absorbent Sheet >

The anti-neck down percentage of the water-absorbent sheet was measured according to the following method. The resulting water-absorbent sheet was cut into a rectangular strip of 5 cm x 20 cm, so that longitudinal direction would be a length direction (machine feeding direction) of the nonwoven fabric. Next, three marking lines of 5 cm in length were drawn with a pencil in a width direction (intersecting direction), with 5 cm intervals in the length direction, to prepare a test piece for the measurement, and the length in the intersection direction was measured at the central marking line was measured (La).
A test piece was fastened to a tensile test machine (manufactured by Shimadzu Corporation, Autograph AGS-J) with a distance between chucks of 10 cm so that the longitudinal direction would be a stretching direction, and stretched at a stretching speed of 10 cm/min at a point where the test piece was stretched to a distance between the chucks of 12 cm, and the machine was stopped for one minute. Thereafter, 5 minutes after the test piece was removed from the chucks, and the length in an intersection direction (Lb) was again measured at the central marking line.

The anti-neck down percentage (N) was calculated according to the following formula. Here, the more the anti-neck down percentage approximates 100, it is judged as an excellent water-absorbent sheet which hardly deforms and quickly recovers from the deformation.

$$N(\%) = Lb/La \times 100$$

< Feel of Water-Absorbent Sheet >

The feel of a water-absorbent sheet was evaluated by the following method. A water-absorbent sheet obtained was cut into a size of 10 cm x 30 cm. Ten panelists were selected, and the panelists were asked to make a three-rank evaluation for feel in accordance with the following criteria, evaluation scores of the panelists were averaged to evaluate the feel of a water-absorbent sheet.

Rank A: The feel of the surface is smooth and soft, and the feel upon bending is also soft (evaluation score: 5).
Rank B: The feel of the surface is smooth, and there is a feel of resistance upon bending (evaluation score: 3).
Rank C: The surface has a roughened feel, is less easier to bend, and has poorer recoverability after bending (evaluation score: 1).

< Evaluations of Total Permeation Rate and Amount of Re-wet of Water-Absorbent Sheet >

A rectangular strip of a water-absorbent sheet of 10 cm x 30 cm, cut in a manner that a longitudinal direction thereof is to be in a length direction (machine feeding direction) of the nonwoven fabric, was used as a sample.

In a 10 L container were placed 60 g of sodium chloride, 1.8 g of calcium chloride dihydrate, 3.6 g of magnesium chloride hexahydrate, and a proper amount of distilled water to completely dissolve. Next, 15 g of an aqueous 1% by mass poly(oxyethylene) isooctylphenyl ether solution was added thereto, and distilled water was further added to adjust the weight of the overall aqueous solution to 6000 g. Thereafter, the mixed solution was colored with a small amount of Blue No. 1 to prepare a test solution.

A polyethylene air-through style porous liquid-permeable sheet having the same size as the sample (10 cm x 30 cm) and a basis weight of 22 g/m$^2$ was placed over an upper side of a sample (water-absorbent sheet). In addition, underneath the sample was placed a polyethylene liquid-impermeable sheet having the same size and basis weight as the sheet, to prepare a simple absorbent article. A cylindrical cylinder having an inner diameter of 3 cm was placed near the central section of this absorbent article, and a 50 mL test solution was supplied thereto at one time. At the same time, a time period until the test solution was completely permeated into the absorbent article was measured with a stopwatch, which is referred to as a first permeation rate (sec). Next, the same procedures were carried out 30 minutes thereafter and 60 minutes thereafter, to measure second and third permeation rates (sec). A total of the number of seconds for the first to third permeation rates is referred to as a total permeation rate.

After 120 minutes from the start of the feeding of the first test liquid, the cylinder was removed, filter papers of 10 cm each side, of which mass was previously measured (We (g), about 70 g), were stacked near the liquid supplying position of the absorbent article, and a 5 kg weight having a size of 10 cm on every side was placed thereon. After 5 minutes of applying a load, the mass (Wf (g)) of the filter papers was measured, and an increased mass was defined as the amount of re-wet (g) as follows.

$$\text{Amount of Re-wet (g)} = Wf - We$$

< Slope Leakage Test >

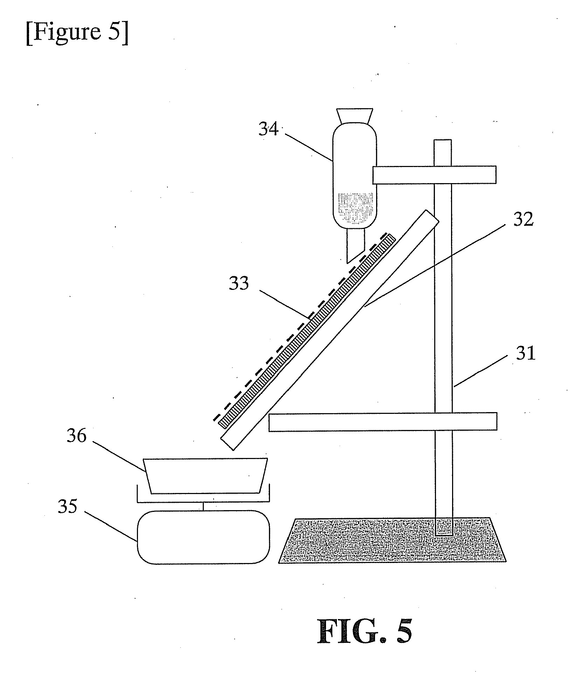

A slope leakage test was conducted using an apparatus shown in Figure 5.
Schematically, a mechanism is as follows. A commercially available stand 31 for experimental facilities was used to slope an acrylic plate 32 and fixed, the above-mentioned test solution was then supplied to a water-absorbent sheet 33 placed on the plate from a dropping funnel 34 positioned vertically above the sheet, and a leakage amount was measured with a balance 35. The detailed specifications are given hereinbelow.

An acrylic plate 32 has a length in the direction of the slope plane of 45 cm, and fixed so that an angle formed with a stand 31 against the horizontal is 45° $\pm$ 2°. The acrylic plate 32 had a width of about 100 cm and a thickness of about 1 cm, and plural water-absorbent sheets 33 could be concurrently measured. The acrylic plate 32 had a smooth surface, so that a liquid was not detained or absorbed to the plate.

A dropping funnel 34 was fixed at a position vertically above the sloped acrylic plate 32 using the stand 31. The dropping funnel 34 had a volume of 100 mL, and an inner diameter of a tip end portion of about 4 mm$\phi$, and an aperture of the cock was adjusted so that a liquid was supplied at a rate of 8 mL/seconds.

A balance 35 on which a metallic tray 36 was placed was set at a lower side of the acrylic plate 32, and all the test solutions flowing down the acrylic plate was received as leakage, and its mass was recorded to the accuracy of 0.1 g.

A slope leakage test using an apparatus as described above was carried out in accordance with the following procedures. The mass of a water-absorbent sheet 33 cut into a rectangular strip of a length of 30 cm and a width of 10 cm in a manner that the longitudinal direction is a length direction (machine feeding direction) of the nonwoven fabric was measured, and an air through-style polyethylene liquid permeable nonwoven fabric (basis weight: 22 g/m$^2$) of the same size was attached from an upper side thereof, and further a polyethylene liquid impermeable nonwoven fabric having the same basis weight of the same size was attached from a lower side thereof to prepare a simple absorbent article. The simple absorbent article was adhered on the acrylic plate 32 (in order not to stop leakage intentionally, the bottom end of the water-absorbent sheet 33 was not adhered to the acrylic plate 32).

Marking was put on the water-absorbent sheet 33 at a position 2 cm away in a downward direction from a top end thereof, and a supplying inlet for the dropping funnel 34 was fixed so that the inlet was positioned at a distance 8 mm $\pm$ 2 mm vertically above the marking.

A balance 35 was turned on, and tared so that the indication was zero, and thereafter 80 mL of the above-mentioned test solution was supplied at one time to the dropping funnel 34. An amount of liquid poured into a metallic tray 36 after the test solution was allowed to flow over a sloped acrylic plate 32 without being absorbed into a water-absorbent sheet 33 was measured, and this amount of liquid is referred to a first leakage amount (mL). The numerical value for this first leakage amount (mL) is denoted as LW1.

Second and third test solutions were supplied in 10-minute intervals from the beginning of the first supply, and second and third leakage amounts (mL) were measured, and the numerical values therefor are respectively denoted as LW2 and LW3.

Next, a leakage index was calculated in accordance with the following equation. The more the index approaches to zero, the smaller the leakage amount at a slope of a water-absorbent sheet, especially an initial leakage amount, whereby it is judged to be an excellent water-absorbent sheet.

$$\text{Leakage Index:} \quad L = LW1 \times 10 + LW2 \times 5 + LW3$$

< Degree of Hydrophilicity of Nonwoven Fabric >

In the present specification, the degree of hydrophilicity of the nonwoven fabrics was measured using an apparatus described in "Determination of Water Repellency" described in JAPAN TAPPI Test Method No. 68 (2000).

Specifically, a nonwoven fabric test piece, which was cut into a rectangular strip of a length of 30 cm and a width of 10 cm in a manner so that the longitudinal direction was a length direction (machine feeding direction) of the nonwoven fabric, was attached to a test piece attaching apparatus sloped at 45°. A burette controlled at an opening of a cock of the burette so that 10 g of distilled water was supplied in 30 seconds was once dried, and fixed so that a part 5 mm above in a vertical direction from the uppermost part of a test piece attached to the sloped apparatus was arranged at the tip of the burette. About 50 g of distilled water was added from the upper part of the burette, and a time period (sec) from the beginning of dripping of a liquid to a nonwoven fabric from the tip of the burette to a point where the liquid leaked out from a lower part because the test piece could not hold on to the liquid was measured, and defined as a degree of hydrophilicity of a nonwoven fabric. It is judged that the larger the numerical values, the higher the degree of hydrophilicity.

Usually, the material itself of the nonwoven fabric having hydrophilicity or a nonwoven fabric provided with a hydrophilic treatment has a numerical value for a degree of hydrophilicity of 5 or more, while in a nonwoven fabric of a material having a low hydrophilicity, liquid droplets are more likely to run smoothly over the surface and leak out from a lower part immediately.

(Example 1)

A spunbond-meltblown-spunbond (SMS) nonwoven fabric made of polypropylene having a width of 30 cm hydrophilically treated with a hydrophilic treatment agent (basis weight: 13 g/m$^2$, thickness: 150 $\mu$m, polypropylene

content: 100%, degree of hydrophilicity: 16, referred to as "Nonwoven Fabric A-1") was spread over a hot melt applicator (manufactured by HALLYS Corporation, Marshall 150) of which heating temperature was set at 150°C, and thereafter a styrene-butadiene-styrene block copolymer (SBS, softening point: 85°C) was coated as an adhesive over the nonwoven fabric at a basis weight of 15 g/m$^2$.

Next, a roller spreader (manufactured by HASHIMA CO., LTD., SINTERACE M/C) was charged at its supplying inlet with a sodium polyacrylate crosslinked product (manufactured by Sumitomo Seika Co., Ltd., AQUAKEEP SA55SX-II, median particle size: 360 $\mu$m; water-absorption rate of saline solution: 42 seconds, water retention capacity of saline solution: 35 g/g, initial water-absorption rate: 0.18 mL/s, effective amount of water absorbed: 58 mL/g, referred to as "Resin A") as a water-absorbent resin. On the other hand, the above-mentioned nonwoven fabric coated with an adhesive was spread over a conveyor at the bottom side of the spreader. Subsequently, the spreading roller and the bottom side conveyor were operated, thereby allowing Resin A to evenly overlay over the nonwoven fabric at a basis weight of 200 g/m$^2$.

The laminate obtained was pressed from a top side with another nonwoven fabric A-1 as a breathable fractionating layer to which the above-mentioned SBS was applied as an adhesive in the same manner as above at a basis weight of 15 g/m$^2$, and thereafter heat-fused with a thermal laminating machine (manufactured by HASHIMA CO., LTD., straight linear fusing press HP-600LF) set at 100°C to integrate, to give an intermediate product of a water-absorbent sheet.

The intermediate product of a water-absorbent sheet was spread over the hot melt applicator of which heating temperature was set at 150°C in the same manner as described above, so that a breathable fractionating layer would be positioned on top, and thereafter the above-mentioned SBS as an adhesive was applied to the intermediate product of a water-absorbent sheet at a basis weight of 10 g/m$^2$.

Next, the roller spreader was charged at its supplying inlet with a sodium polyacrylate crosslinked product (manufactured by Sumitomo Seika Co., Ltd., AQUAKEEP 10SH-PB, median particle size: 320 $\mu$m; water-absorption rate of saline solution: 3 seconds, water retention capacity of saline solution: 42 g/g, effective amount of water absorbed: 65 mL/g, referred to as "Resin B") as a water-absorbent resin. On the other hand, the intermediate product of a water-absorbent sheet was spread over a conveyor at the bottom side of the spreader, with the side coated with an adhesive on top. Subsequently, the spreading roller and the bottom side conveyor were operated, thereby allowing Resin B to evenly overlay over the above-mentioned nonwoven fabric made of the above-mentioned intermediate product of a water-absorbent sheet at a basis weight of 50 g/m$^2$.

The laminate obtained was pressed from a top side with another nonwoven fabric A-1 to which the above-mentioned SBS was applied in the same manner as above as an adhesive at a basis weight of 10 g/m$^2$, and thereafter heat-fused with a thermal laminating machine (manufactured by HASHIMA CO., LTD., straight linear fusing press HP-600LF) set at 100°C to integrate, to give a water-absorbent sheet. The cross section of the structure of the water-absorbent sheet obtained had a structure as schematically shown in Figure 3.

The water-absorbent sheet obtained was cut into a given size to measure the properties of the water-absorbent sheet. The results are shown in Table 2.

(Examples 2 to 4)

The same procedures as in Example 1 were carried out except that in Example 1, the kinds of the nonwoven fabric used were changed to those shown in Table 1, to give each of water-absorbent sheets. The water-absorbent sheet obtained was cut into a given size to measure the properties of the water-absorbent sheet. The results are shown in Table 2.

(Example 5)

A roller spreader (manufactured by HASHIMA CO., LTD., SINTERACE M/C) was charged at its supplying inlet with a mixture prepared by homogeneously mixing 40 parts by mass of a low-density polyethylene (melting temperature: 107°C) as an adhesive and 350 parts by mass of Resin A as a water-absorbent resin. On the other hand, an SMS nonwoven fabric made of polypropylene having a width of 30 cm hydrophilically treated with a hydrophilic treatment agent (basis weight: 11 g/m$^2$, thickness: 120 $\mu$m, polypropylene content: 100%, degree of hydrophilicity: 12, referred to as "Nonwoven Fabric A-4") was spread over a conveyor at a bottom side of the roller spreader. Subsequently, the spreading roller and the bottom side conveyor were operated, thereby allowing the above-mentioned mixture to evenly overlay over the above-mentioned nonwoven fabric at a basis weight of 390 g/m$^2$.

The laminate obtained was pressed with another nonwoven fabric A-4 as a breathable fractionating layer, and heat-fused with a thermal laminating machine (manufactured by HASHIMA CO., LTD., straight linear fusing press HP-600LF) set at 140°C to integrate, to give an intermediate product of a water-absorbent sheet.

Separately, a roller spreader (manufactured by HASHIMA CO., LTD., SINTERACE M/C) was charged at its supplying inlet with a mixture prepared by homogeneously mixing 30 parts by mass of the above-mentioned low-density polyethylene as an adhesive and 80 parts by mass of Resin B as a water-absorbent resin. The above-mentioned intermediate product of a water-absorbent sheet was spread over a conveyor of the spreader, with the side of a breathable fractionating layer on top, and the spreading roller and the bottom side conveyor were operated, thereby allowing the above-mentioned mixture to evenly overlay over the above-mentioned intermediate product at a basis weight of 110 g/m$^2$.

The laminate obtained was pressed from a top side with another nonwoven fabric A-4, and heat-fused with a thermal laminating machine (manufactured by HASHIMA CO., LTD., straight linear fusing press HP-600LF) set at 140°C to integrate, to give a water-absorbent sheet. The cross section of the structure of the resulting water-absorbent sheet, as schematically shown, had a structure as shown in Figure 1. The water-absorbent sheet obtained was cut into a given size to measure the properties of the water-absorbent sheet. The results are shown in Table 2.

(Comparative Example 1)

A meltblown nonwoven fabric made of polypropylene having a width of 30 cm (basis weight: 15 g/m$^2$, thickness: 120 $\mu$m, polypropylene content: 100%, degree of hydrophilicity: 15, referred to as "Nonwoven Fabric C") was spread over a hot melt applicator (manufactured by HALLYS Corporation, Marshall 150) of which heating temperature was set at 150°C, and thereafter the same SBS as in Example 1 was coated as an adhesive over the nonwoven fabric at a basis weight of 20 g/m$^2$.

Next, a roller spreader (manufactured by HASHIMA CO., LTD., SINTERACE M/C) was charged at its supplying inlet with Resin A as a water-absorbent resin. On the other hand, a nonwoven fabric coated with an adhesive was spread over a conveyor at the bottom side of the spreader. Subsequently, the spreading roller and the bottom side conveyor were operated, thereby allowing Resin A to evenly overlay over the nonwoven fabric at a basis weight of 250 g/m$^2$.

The laminate obtained was pressed from a top side with the nonwoven fabric C to which the above-mentioned SBS was applied in the same manner as above at a basis weight of 20 g/m$^2$, and thereafter heat-fused with a thermal laminating machine (manufactured by HASHIMA CO., LTD., straight linear fusing press HP-600LF) of which heating temperature was set at 100°C to integrate, to give a comparative water-absorbent sheet. The water-absorbent sheet was cut into a given size to measure the properties of the water-absorbent sheet. The results are shown in Table 2.

(Comparative Examples 2 to 4)

The same procedures as in Example 1 were carried out except that in Example 1, the kinds of the nonwoven fabric used and the like were changed to those shown in Table 1, to give each of water-absorbent sheets. The water-absorbent sheet obtained was cut into a given size to measure the properties of the water-absorbent sheet. The results are shown in Table 2.

[Table 1]

Table 1

| Ex. No. | Nonwoven Fabrics | | | | | | Water-Absorbent Resin (g/m²) | | | | | Adhesive (g/m²) | | | |
|---|---|---|---|---|---|---|---|---|---|---|---|---|---|---|---|
| | Upper Side | g/m² | Breathable Fractionating Layer | g/m² | Lower Side | g/m² | Primary | | Secondary | | Ratio* | Kinds | Primary | Secondary | Content** |
| Ex. 1 | Nonwoven Fabric A-1 | 13 | Nonwoven Fabric A-1 | 13 | Nonwoven Fabric A-1 | 13 | Resin A | 200 | Resin B | 50 | 81/19 | SBS | 30 | 20 | 0.20 |
| Ex. 2 | Nonwoven Fabric A-2 | 18 | Nonwoven Fabric A-2 | 18 | Nonwoven Fabric A-2 | 18 | Resin A | 150 | Resin A | 50 | 75/25 | SBS | 20 | 20 | 0.20 |
| Ex. 3 | Nonwoven Fabric B-1 | 34 | Nonwoven Fabric A-1 | 13 | Nonwoven Fabric A-1 | 13 | Resin A | 150 | Resin B | 150 | 50/50 | SBS | 30 | 30 | 0.20 |
| Ex. 4 | Nonwoven Fabric A-3 | 15 | Nonwoven Fabric A-3 | 15 | Nonwoven Fabric A-3 | 15 | Resin A | 250 | Resin B | 50 | 83/17 | SBS | 30 | 10 | 0.13 |
| Ex. 5 | Nonwoven Fabric A-4 | 11 | Nonwoven Fabric A-4 | 11 | Nonwoven Fabric A-4 | 11 | Resin A | 350 | Resin B | 80 | 81/19 | Polyethylene | 40 | 30 | 0.16 |
| Comp. Ex.1 | Nonwoven Fabric C | 15 | - | - | Nonwoven Fabric C | 15 | Resin A | 250 | - | - | - | SBS | 40 | - | 0.16 |
| Comp. Ex. 2 | Nonwoven Fabric A-1 | 13 | Nonwoven Fabric A-1 | 13 | Nonwoven Fabric A-5 | 17 | Resin A | 200 | Resin B | 50 | 81/19 | SBS | 30 | 20 | 0.20 |
| Comp. Ex. 3 | Nonwoven Fabric A-5 | 17 | Nonwoven Fabric A-5 | 17 | Nonwoven Fabric A-5 | 17 | Resin A | 200 | Resin B | 50 | 81/19 | SBS | 30 | 20 | 0.20 |
| Comp. Ex. 4 | Nonwoven Fabric B-2 | 40 | Nonwoven Fabric B-2 | 40 | Nonwoven Fabric B-2 | 40 | Resin A | 200 | Resin B | 50 | 81/19 | SBS | 30 | 20 | 0.20 |

*: Ratio of primary absorbent layer to secondary absorbent layer, i.e. primary/secondary, of water-absorbent resin (mass ratio)

**: Content of adhesive (content based on the water-absorbent resin (mass basis))

Here, Resin A and Resin B are resins obtained by a reversed phase suspension polymerization method.
[Table 2]

Table 2

| Ex. No. | Thickness (mm) | Permeation Rate (sec) | | | | Amount of Rewet (g) | Slope Leakage Test | | | | Anti-Neck Down Percentage of Water-Absorbent Sheet (%) | Feel of Water-Absorbent Sheet |
|---|---|---|---|---|---|---|---|---|---|---|---|---|
| | | 1 | 2 | 3 | Total | | 1 | 2 | 3 | Index | | |
| Ex. 1 | 1.1 | 36 | 15 | 17 | 68 | 15.0 | 2 | 0 | 0 | 20 | 95 | 4.3 |
| Ex. 2 | 1.2 | 37 | 16 | 18 | 71 | 16.0 | 3 | 0 | 0 | 30 | 97 | 4.4 |
| Ex. 3 | 1.2 | 41 | 22 | 22 | 85 | 14.0 | 1 | 0 | 0 | 10 | 95 | 4.9 |
| Ex. 4 | 1.2 | 38 | 17 | 19 | 74 | 7.0 | 1 | 0 | 0 | 10 | 96 | 4.3 |
| Ex. 5 | 1.3 | 35 | 22 | 20 | 77 | 3.0 | 2 | 0 | 0 | 20 | 95 | 4.2 |
| Comp. Ex. 1 | 1.1 | 44 | 26 | 32 | 102 | 18.0 | 15 | 1 | 0 | 155 | 84 | 2.6 |
| Comp. Ex. 2 | 1.1 | 42 | 23 | 26 | 91 | 21.0 | 10 | 4 | 0 | 120 | 95 | 4.5 |
| Comp. Ex. 3 | 1.2 | 50 | 25 | 18 | 93 | 10.0 | 65 | 65 | 40 | 1015 | 96 | 4.3 |
| Comp. Ex. 4 | 1.7 | 38 | 21 | 25 | 84 | 7.4 | 1 | 0 | 0 | 10 | 79 | 4.8 |

Further, Table 3 shows the details on structures, materials and the like of the nonwoven fabrics used in each of Examples and Comparative Examples. Here, the rayon percentages in the materials (rayon, PET) for the nonwoven fabrics B-1 and B-2 were 70%, respectively.
[Table 3]

| Abbreviation | Structure | Material | Basis Weight g/m$^2$ | Thickness $\mu$m | Degree of Hydrophilicity |
|---|---|---|---|---|---|
| Nonwoven Fabric A-1 | SMS | Polypropylene | 13 | 150 | 16 |
| Nonwoven Fabric A-2 | SMS | Polypropylene | 18 | 200 | 24 |
| Nonwoven Fabric A-3 | SMMS | Polypropylene | 15 | 170 | 20 |
| Nonwoven Fabric A-4 | SMS | Polypropylene | 11 | 120 | 12 |
| Nonwoven Fabric A-5 | SMS | Polypropylene | 17 | 190 | 3 or less |
| Nonwoven Fabric B-1 | Spunlace | Rayon, PET | 34 | 350 | 40 |
| Nonwoven Fabric B-2 | Spunlace | Rayon, PET | 40 | 400 | 55 |
| Nonwoven Fabric C | Meltblown | Polypropylene | 15 | 120 | 15 |

It could be seen from Tables 1 and 2 that the water-absorbent sheets having the structures of the present invention using a specified nonwoven fabric as in Examples 1 to 5 have excellent properties in the permeation rates, the amount of re-wet, the slope leakage index, and the like. Further, when the internal portion of the water-absorbent sheet after carrying out the slope leakage test, the absorbent layer was evenly swollen in its entirety, so that the gel blocking phenomenon did not take place.

On the other hand, when the comparative examples are studied, in a case where an absorbent layer is a single layer and only a meltblown nonwoven fabric is used (Comparative Example 1), the evaluations of both the total permeation rate and the leakage index were low, and the water-absorbent sheets had poorer anti-neck down percentage and feel. Even when all of the two nonwoven fabrics sandwiching the absorbent layer and the breathable fractionating layer are spunbond nonwoven fabrics, in a case where a nonwoven fabric at a lower side is hydrophobic (Comparative Examples 2 and 3), the absorbent properties were not sufficient. In particular, the slope leakage indexes were likely to be high, so that it was predicted to have a possibility of leading to liquid leakage when absorbent articles were prepared therefrom. It could be seen from these that in a case where at least a nonwoven fabric at a lower side does not have hydrophilicity, the absorbent properties as the water-absorbent sheet of the level that can be used for absorbent articles would no way be reached. In a case where rayon spunlace nonwoven fabrics having hydrophilicity were used for the above-mentioned three sheets of nonwoven fabrics (Comparative Example 4), although the absorbent properties were at a level that can be used, the anti-neck down percentage of the water-absorbent sheet was low; therefore, in a case where the water-absorbent sheet was stretched in a machine feeding direction in the production steps of the absorbent article, variances in lengths in an intersection direction are caused, so that there is a risk of making the quality of the absorbent article unstable, so that it cannot be said to be sufficiently satisfactory as a water-absorbent sheet.

## INDUSTRIAL APPLICABILITY

The water-absorbent sheet of the present invention can be used for absorbent articles in hygienic material fields, agricultural fields, construction material fields, and the like, among which the water-absorbent sheet can be suitably used for absorbent articles in the hygienic material fields.

## EXPLANATION OF NUMERICAL SYMBOLS

| | |
|---|---|
| 1 | burette section |
| 2 | lead tube |
| 3 | measuring platform |
| 4 | nonwoven fabric |
| 5 | water-absorbent resin |
| 6 | stand |
| 7 | clamp |
| 10 | burette |
| 11 | air inlet tube |
| 12 | cock |
| 13 | cock |
| 14 | rubber plug |
| 31 | stand |
| 32 | acrylic plate |
| 33 | water-absorbent sheet |
| 34 | dropping funnel |
| 35 | balance |
| 36 | metallic tray |
| 50 | adhesives |
| 51 | water-absorbent sheet |
| 52 | water-absorbent resin |
| 53 | primary absorbent layer |
| 54 | water-absorbent resin |
| 55 | secondary absorbent layer |
| 56 | breathable fractionating layer |
| 57 | nonwoven fabric |
| 58 | hydrophilic nonwoven fabric |

59    other nonwoven fabric
60    adhesive

**Claims**

1. A water-absorbent sheet comprising a structure in which an absorbent layer comprising a water-absorbent resin and an adhesive is sandwiched with nonwoven fabrics from an upper side and a lower side of the absorbent layer, wherein the absorbent layer has a laminate structure that is fractionated into a primary absorbent layer and a secondary absorbent layer with a breathable fractionating layer, and wherein at least one sheet of the nonwoven fabrics is a spunbond nonwoven fabric, and at least a nonwoven fabric at a lower side of the nonwoven fabrics is a hydrophilic nonwoven fabric.

2. The water-absorbent sheet according to claim 1, wherein the spunbond nonwoven fabric comprises fibers selected from the group consisting of polyolefins, polyesters, and blends thereof.

3. The water-absorbent sheet according to claim 1 or 2, wherein the nonwoven fabric has a basis weight of 35 g/m$^2$ or less.

4. The water-absorbent sheet according to any one of claims 1 to 3, wherein at least one sheet of the nonwoven fabrics other than the spunbond nonwoven fabric is a nonwoven fabric made of one or more fibers selected from the group consisting of rayon fibers, rayon fibers/polyolefin fibers, and rayon fibers/polyester fibers.

5. The water-absorbent sheet according to any one of claims 1 to 4, wherein the breathable fractionating layer is formed by a nonwoven fabric.

6. The water-absorbent sheet according to any one of claims 1 to 5, wherein the water-absorbent resin is contained per 1 m$^2$ of the water-absorbent sheet in an amount of from 100 to 1000 g, and wherein the adhesive is contained in an amount of 0.05 to 2.0 times the amount of the water-absorbent resin contained.

7. The water-absorbent sheet according to any one of claims 1 to 6, wherein the adhesive is one or more members selected from the group consisting of polyolefin-based adhesives, polyester-based adhesives, ethylene-vinyl acetate copolymer adhesives, and styrene-based elastomer-based adhesives.

8. The water-absorbent sheet according to any one of claims 1 to 7, wherein the water-absorbent sheet satisfies all the properties of the following (A) to (C):

    (A) the water-absorbent sheet having a thickness of 3 mm or less,
    (B) a total permeation rate of 100 seconds or less, and
    (C) a leakage index of 100 or less.

9. An absorbent article comprising the water-absorbent sheet as defined in any one of claims 1 to 8, sandwiched between a liquid-permeable sheet and a liquid-impermeable sheet.

[Figure 1]

57
51
52
50
56
50
54
53
58
55

## FIG. 1

[Figure 2]

59
51
52
50
56
50
54
53
58
55

## FIG. 2

[Figure 3]

FIG. 3

[Figure 4]

**FIG. 4**

[Figure 5]

**FIG. 5**

## INTERNATIONAL SEARCH REPORT

| | International application No. |
|---|---|
| | PCT/JP2010/067225 |

A. CLASSIFICATION OF SUBJECT MATTER
*A61F13/15(2006.01)i, A61F13/49(2006.01)i, A61F13/53(2006.01)i*

According to International Patent Classification (IPC) or to both national classification and IPC

B. FIELDS SEARCHED

Minimum documentation searched (classification system followed by classification symbols)
A61F13/15, A61F13/49, A61F13/53

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2010 |
| Kokai Jitsuyo Shinan Koho | 1971–2010 | Toroku Jitsuyo Shinan Koho | 1994–2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2001-46435 A  (Oji Paper Co., Ltd.),<br>20 February 2001 (20.02.2001),<br>claims; paragraphs [0014] to [0027], [0034];<br>fig. 1, 8<br>(Family: none) | 1-9 |
| Y | JP 6-315501 A  (Sekisui Plastics Co., Ltd.),<br>15 November 1994 (15.11.1994),<br>claim 3; paragraphs [0018], [0048] to [0057];<br>fig. 5 to 6<br>(Family: none) | 1-9 |

| ☒ | Further documents are listed in the continuation of Box C. | ☐ | See patent family annex. |
|---|---|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 December, 2010 (13.12.10) | 21 December, 2010 (21.12.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/067225

| C (Continuation). DOCUMENTS CONSIDERED TO BE RELEVANT | | |
|---|---|---|
| Category* | Citation of document, with indication, where appropriate, of the relevant passages | Relevant to claim No. |
| Y | JP 2002-325799 A (Japan Absorbent Technology Institute), 12 November 2002 (12.11.2002), claims; paragraphs [0018] to [0019], [0025] to [0026]; fig. 1 (Family: none) | 4-9 |
| A | JP 2002-224161 A (Mitsubishi Chemical Corp.), 13 August 2002 (13.08.2002), paragraphs [0007], [0010] to [0013], [0019]; fig. 1 (Family: none) | 1-9 |
| A | Microfilm of the specification and drawings annexed to the request of Japanese Utility Model Application No. 153083/1987(Laid-open No. 58607/1989) (Shiseido Co., Ltd.), 12 April 1989 (12.04.1989), entire text; all drawings (Family: none) | 1-9 |
| A | JP 2006-513823 A (The Procter & Gamble Co.), 27 April 2006 (27.04.2006), entire text; all drawings & JP 2007-222646 A & JP 2007-244882 A & JP 2007-244883 A & US 2004/0162536 A1 & US 2007/0156108 A1 & US 2007/0167928 A1 & US 2007/0179464 A1 & US 2010/0228211 A & EP 1447066 A1 & EP 1808152 A2 & EP 1813236 A2 & EP 1813237 A2 & EP 1982678 A1 & WO 2004/071363 A1 | 1-9 |
| P,A | WO 2010/076857 A1 (Sumitomo Seika Chemicals Co., Ltd.), 08 July 2010 (08.07.2010), entire text; all drawings (Family: none) | 1-9 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI9510889 B
- JP HEI8057311 B
- JP 2000238161 A
- JP HEI7051315 B
- JP 2002325799 A
- JP 2003011118 A
- JP HEI02048944 B

**Non-patent literature cited in the description**

- Determination of Water Repellency. *JAPAN TAPPI Test Method No. 68,* 2000